# EUROPÄISCHE PATENTSCHRIFT

(11) **EP 1 285 641 B1**
(45) Veröffentlichungstag und Bekanntmachung des Hinweises auf die Patenterteilung: **11.05.2005**
(21) Anmeldenummer: 02014817.7
(22) Anmeldetag: 02.07.2002
(51) Int. Cl.: A61F 5/453

(54) **Vorrichtung zur Ableitung von unkontrolliertem Harnabgang bei männlichen Personen**
Device to drain away uncontrolled male urination
Dispositif pour écouler de l'urine non-contrôlée chez l'homme

(30) Priorität: 13.08.2001 DE 20113445 U
(43) Veröffentlichungstag der Anmeldung: 26.02.2003
(73) Patentinhaber: Grundke, Reinhold, 84489 Burghausen (DE); Liedl, Bernhard, Dr. med., 81337 München (DE); Jaeniche, Wilhelm, 77694 Kehl-Leutesheim (DE)
(72) Erfinder: Grundke, Reinhold, 84489 Burghausen (DE); Liedl, Bernhard, Dr. med., 81337 München (DE); Jaeniche, Wilhelm, 77694 Kehl-Leutesheim (DE)
(74) Vertreter: Hano, Christian, Dipl.-Ing.

(56) Entgegenhaltungen:
- EP-A- 0 824 905
- WO-A-00/51532
- FR-A- 2 704 427
- US-A- 2 940 450
- US-A- 5 084 037
- US-A- 5 713 880
- US-A- 5 830 932

## Beschreibung

Die Erfindung betrifft eine Vorrichtung zur Ableitung von unkontrolliertem Harnabgang bei männlichen Personen mit einem Grundkörper, der einen Kammerabschnitt aufweist, an den im vorderen Bereich ein sich nach vorne verjüngender Trichterabschnitt anschließt, der in einen Schlauchanschluss übergeht, wobei im rückwärtigen Bereich des Grundkörpers Einrichtungen zur Befestigung der Vorrichtung am Penis eines Benutzers vorgesehen sind.

Aus der EP 0 824 905 A1 ist eine Vorrichtung zur Ableitung von unkontrolliertem Harnabgang bei männlichen Personen bekannt, die einen Grundkörper aus Weichplastik aufweist. Der mittlere Abschnitt des Grundkörpers bildet einen zylindrischen, auf den Penisquerschnitt abgestimmten Mantel, an den sich an einem Ende eine sich trichterförmig in Richtung auf einen Schlauchanschluss verengende Trichterkammer anschließt. Der Mantel ist am anderen Ende durch in Umfangsrichtung aufeinanderfolgende Unterbrechungen in mehrere Zungen unterteilt. Außerdem ist am Grundkörper am Übergang vom geschlossenen Mantel zu den Zungen eine weichelastische, schmiegsame Manschette anbringbar, die geeignet ist, die Zungen von außen elastisch zu umschließen. Am Mantel ist eine sich nach außen erstreckende Anschlussstelle für eine Spülvorrichtung vorgesehen.

Es hat sich gerade bei älteren Menschen mit schlechter Motorik gezeigt, daß die Anbringung der bekannten Vorrichtung aufgrund der Zweiteiligkeit zu kompliziert ist, so daß die Vorrichtung häufig schlecht sitzend angebracht wurde. Außerdem kann es vorkommen, daß Harn durch die Unterbrechungen zwischen zwei benachbarten Zungen austreten kann.

US-A-5 713 880 Figur 8 zeigt eine ähnliche Vorrichtung jedoch ohne Dehnzungen und aus Latexmaterial.

Der Erfindung liegt die Aufgabe zugrunde, mit konstruktiv einfachen Mitteln eine Vorrichtung zur Ableitung von unkontrolliertem Harnabgang bei männlichen Personen zu schaffen, die leicht angebracht werden kann und gewährleistet, dass kein Harn am rückwärtigen Ende austreten kann.

Diese Aufgabe wird durch eine Vorrichtung mit den Merkmalen des Patentanspruchs 1 gelöst. Vorteilhafte Weiterbildungen der erfindungsgemäßen Vorrichtung sind Gegenstand der Patentansprüche 2 bis 4.

Die erfindungsgemäße Vorrichtung ist leicht am Penis eines Benutzers anbringbar. Hierzu wird jeweils mit einer Hand eine Dehnzunge ergriffen und der Abschlussabschnitt so weit auseinander gedehnt, bis der Penis durch den Abschlussabschnitt in den Grundkörper eingeführt werden kann. Nach Einführung werden die Dehnzungen losgelassen, wodurch sich der Abschlussabschnitt an den Schaft des Penis harndicht anschmiegt. Das Silikonkautschuk-Material ist im Bereich des Abschlussabschnittes so ausgelegt, daß kein Harn austreten kann, aber auch kein Blutstau verursacht wird. Da die Vorrichtung aus Silikonkautschuk besteht, ist sie leicht reinigbar und löst keine Allergien aus.

Bei einer besonderen Ausführungsform ist ein rohrförmiger Spül- und Belüftungsanschluss im Bereich des Übergangsabschnittes vorgesehen, dessen äußere freie Stirnseite innerhalb der gedachten Verlängerung der Außenfläche des Kammerabschnitts liegt. Hierdurch wird ein Scheuern durch den Spül- und Belüftungsanschluss verhindert.

Vorzugsweise sind zwei Dehnzungen diametral gegenüberliegend an dem rückwärtigen Ende des Abschlussabschnittes vorgesehen.

Darüber hinaus kann auch wenigstens eine Befestigungszunge an dem rückwärtigen Ende des Abschlussabschnittes vorgesehen sein, mittels derer die Vorrichtung an einer Unterhose oder an einem Gürtel befestigt werden kann.

Ein Ausführungsbeispiel der Erfindung wird nachstehend anhand von Zeichnungen näher erläutern. Es zeigen:
- Fig. 1 -: eine perspektivische Ansicht einer Vorrichtung zur Ableitung von unkontrolliertem Harnabgang bei männlichen Personen;
- Fig. 2 -: einen Längsschnitt der Vorrichtung von Fig. 1.

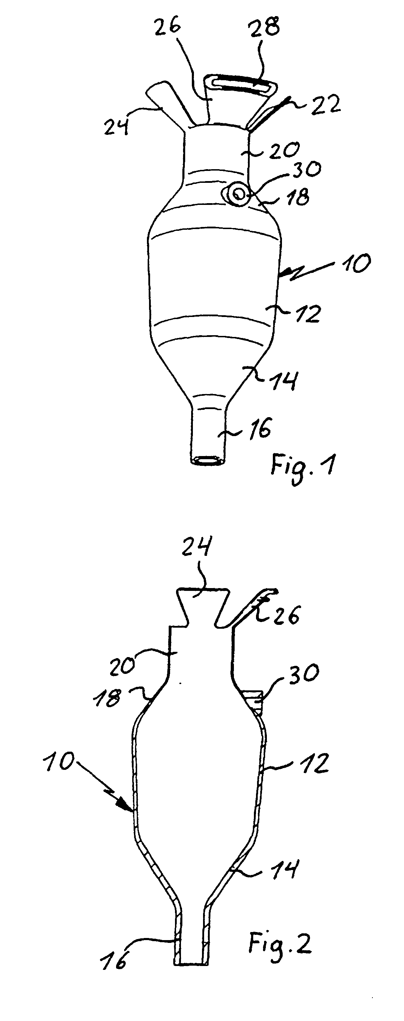

Die Vorrichtung zur Ableitung von unkontrolliertem Harnabgang bei männlichen Personen weist einen einteiligen, rotationssymetrischen Grundkörper 10 aus Silikonkautschuk auf. In seinem mittleren Bereich umfasst der Grundkörper 10 einen zylindrischen Kammerabschnitt 12, dessen Innendurchmesser größer als der Außendurchmesser des Penis eines Benutzers ist. An diesen Kammerabschnitt 12 schließt vorne (in Fig. 1 unten) ein sich nach vorne konisch verjüngender Trichterabschnitt 14 an, der an seinem vorderen Ende in einen Schlauchanschluss 16 übergeht. Auf der dem Trichterabschnitt 14 entgegengesetzten Seite des Kammerabschnitts 12 geht der Kammerabschnitt 12 in einen kegelstumpfförmigen Übergangsabschnitt 18 über, der sich nach hinten (in Fig. 1 nach oben) konisch verjüngt. An diesen Übergangsabschnitt 18 schließt ein zylindrischer Abschlussabschnitt 20 an, dessen Innendurchmesser etwas geringer ist, als der Außendurchmesser des Penis eines Benutzers. Der Ausdruck "etwas geringer" ist so auszulegen, dass der Innendurchmesser z.B. bei einem Penisdurchmesser von 22 bis 40 mm ungefähr 20 mm beträgt. An dem rückwärtigen stirnseitigen Ende des Abschlussabschnittes 20 sind diametral gegenüberliegend zwei Dehnzungen 22, 24 vorgesehen. Zwischen den Dehnzungen 22, 24 ist eine Befestigungszunge 26 angeordnet, die angrenzend an ihr freies Ende eine Durchgangsöse 28 aufweist.

Im Bereich des Übergangsabschnitts 18 ist ein rohrförmiger Spül- und Belüftungsanschluss 30 vorgesehen, der sich radial zur Mittelachse des Grundkörpers 10 erstreckt und mit dem Innenraum des Grundkörpers 10 in Verbindung steht. Die radial äußere Stirnseite des Spül- und Belüftungsanschlusses 30 liegt, wie es in Fig. 2 zu erkennen ist, innerhalb der gedachten Verlängerung der Zylinderaußenfläche des Kammerabschnittes 12.

Zur bestimmungsgemäßen Anbringung der erfindungsgemäßen Vorrichtung auf dem Penis eines Benutzers werden die Dehnzungen 22, 24 ergriffen und diese radial zur Mittelachse des Grundkörpers 10 nach außen gezogen, wodurch sich der Innenquerschnitt des Abschlussabschnittes 20 so weit vergrößert, daß der Penis in den Grundkörper 10 eingeführt werden kann. Anschließend werden die Dehnzungen 22, 24 losgelassen, wodurch sich der Abschlussabschnitt 20 an den Penis mit einer Druckkraft anschmiegt, die einen Austritt von Harn über den Abschlussabschnitt 20 verhindert. Gleichzeitig ist die Druckkraft so ausgelegt, daß kein Blutstau im Penis auftritt.

Anschließend wird die Vorrichtung mittels der der Öse 28 an der Befestigungszunge 26 an einen Gürtel oder dgl. befestigt. Anstatt der Öse 28 kann auch ein Klettband oder dgl. an der Befestigungszunge 26 angebracht werden.

Die Dimensionen der erfindungsgemäßen Vorrichtung können abhängig von der jeweiligen Größe des Penis eines Benutzers ausgelegt werden. Bei einer für den durchschnittlichen Benutzer ausgelegten Vorrichtung beträgt der Innendurchmesser des Kammerabschnitts ungefähr 43 mm. Der Abschlussabschnitt 20 dieser Ausführungsform hat bevorzugt einen Innendurchmesser von 20 mm und einen Außendurchmesser von 21 mm. Die Länge des Abschlussabschnittes 20 beträgt ca. 17,5 mm. Eine solche Ausführungsform ist für einen Penisdurchmesser zwischen 22 und 40 mm geeignet.

## Patentansprüche

1. Vorrichtung zur Ableitung von unkontrolliertem Harnabgang bei männlichen Personen mit einem einteiligen Grundkörper (10) aus Silikonkautschuk, der
- im mittleren Bereich einen Kammerabschnitt (12) aufweist, dessen Innenquerschnitt größer als der Querschnitt des Penis eines Benutzers ist,
- im vorderen Bereich einen an den zylindrischen Kammerabschnitt (12) anschließenden, sich nach vorne verjüngenden Trichterabschnitt (14) aufweist, der in einen Schlauchanschluss (16) übergeht, und
- im rückwärtigen Bereich einen an den Kammerabschnitt (12) anschließenden, sich nach hinten verjüngenden Übergangsabschnitt (18) aufweist, der in einen zylinderförmigen Abschlussabschnitt (20) übergeht, dessen Innendurchmesser etwas geringer ist als der Außendurchmesser des Penis des Benutzers, wobei an dem rückwärtigen Ende des Abschlussabschnittes (20) wenigstens zwei Dehnzungen (22, 24) vorgesehen sind.

2. Vorrichtung nach Anspruch 1, **dadurch gekennzeichnet, dass** im Bereich des Übergangsabschnittes (18) ein rohrförmiger Spül- und Belüftungsanschluss (30) vorgesehen ist, dessen äußere, freie Stirnseite innerhalb der gedachten Verlängerung der Außenfläche des Kammerabschnitts (12) liegt.

3. Vorrichtung nach Anspruch 1 oder 2, **dadurch gekennzeichnet, dass** zwei Dehnzungen (22, 24) diametral gegenüberliegend an dem rückwärtigen Ende des Abschlussabschnittes (20) vorgesehen sind.

4. Vorrichtung nach einem der vorhergehenden Ansprüche, **dadurch gekennzeichnet, dass** an dem rückwärtigen Ende des Abschlussabschnittes (20) wenigstens eine Befestigungszunge (26) vorgesehen ist.

## Claims

1. A device for draining uncontrolled urine release in male persons, with a one-piece body (10) which consists of silicone rubber and which
- in its central region has a chamber portion (12) whose inner cross-section is larger than the penis cross section of a user,
- in its front region has a forwardly tapering funnel portion (14) which adjoins the cylindrical chamber portion (12) and which merges into a hose connection (16), and
- in its rear region has a rearwardly tapering transitional portion (18) which adjoins the chamber portion (12) and which merges into a cylindrical end portion (20) whose inner diameter is slightly smaller than the outer diameter of the penis of a user, wherein at least two expanding tongues (22,24) are provided at the rear end of the end portion (20).

2. A device according to Claim 1, **characterised in that** in the vicinity of the transitional portion (18) there is provided a tubular rinsing and venting connection (30) whose outer free end face lies within the imaginary extension of the outer surface of the chamber portion (12).

3. A device according to Claim 1 or 2, **characterised in that** two expanding tongues (22,24) are provided diametrically opposite at the rear end of the end portion (20).

4. A device according to any one of the preceding Claims, **characterised in that** at least one fastening tongue (26) is provided at the rear end of the end portion (20).

## Revendications

1. Dispositif pour le drainage d'une excrétion urinaire incontrôlée chez des personnes de sexe masculin, comprenant un corps de base unitaire (10) constitué de caoutchouc de silicone, qui présente
- dans sa zone médiane, une partie de chambre (12), dont la section transversale interne est supérieure à la section transversale du pénis d'un utilisateur,
- dans sa zone avant, une partie en forme d'entonnoir (14) se rétrécissant vers l'avant, qui se raccorde à la partie de chambre (12) cylindrique et qui se transforme en un raccord pour tuyau (16), et
- dans sa zone arrière, une partie de transition (18), se rétrécissant vers l'arrière, qui se raccorde à la partie de chambre (12) et qui se transforme en une partie terminale (20) de forme cylindrique dont le diamètre interne est légèrement inférieur au diamètre externe du pénis de l'utilisateur, au moins deux languettes d'extension (22, 24) étant prévues à l'extrémité arrière de la partie terminale (20).

2. Dispositif selon la revendication 1, **caractérisé en ce qu'**on prévoit, dans la zone de la partie de transition (18), un raccord tubulaire de lavage et d'aération (30) dont le côté frontal externe libre est disposé à l'intérieur du prolongement imaginaire de la face externe de la partie de chambre (12).

3. Dispositif selon la revendication 1 ou 2, **caractérisé en ce que** sont prévues deux languettes d'extension (22, 24) diamétralement opposées à l'extrémité arrière de la partie terminale (20).

4. Dispositif selon l'une quelconque des revendications précédentes, **caractérisé en ce qu'**est prévue au moins une languette de fixation (26) à l'extrémité arrière de la partie terminale (20).
